(19) Europäisches Patentamt
European Patent Office
Office européen des brevets

(11) Veröffentlichungsnummer: **0 060 426**
**B1**

# (12) EUROPÄISCHE PATENTSCHRIFT

(45) Veröffentlichungstag der Patentschrift: **23.04.86**

(21) Anmeldenummer: **82101513.8**

(22) Anmeldetag: **27.02.82**

(51) Int. Cl.[4]: **C 07 D 285/12**, C 07 D 263/58, C 07 D 277/68, C 07 D 277/56, C 07 D 285/08, C 07 D 277/34, A 01 N 43/72

## (54) N-(2,2,2-Trifluorethyl)-N-alkyl-azolyloxyessigsäureamide, Verfahren zu ihrer Herstellung und ihre Verwendung als Herbizide sowie neue Zwischenprodukte zu ihrer Herstellung.

(30) Priorität: **13.03.81 DE 3109582**

(43) Veröffentlichungstag der Anmeldung: **22.09.82 Patentblatt 82/38**

(45) Bekanntmachung des Hinweises auf die Patenterteilung: **23.04.86 Patentblatt 86/17**

(84) Benannte Vertragsstaaten: **AT BE CH DE FR GB IT LI NL**

(56) Entgegenhaltungen:
**EP - A - 0 004 187**
**EP - A - 0 005 501**
**EP - A - 0 018 497**
**EP - A - 0 029 171**
**EP - A - 0 037 525**
**EP - A - 0 037 938**
**EP - A - 0 044 497**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Mues, Volker, Dr., Gellertweg 13, D-5600 Wuppertal 1 (DE)**
Erfinder: **Baasner, Bernd, Dr., Kalkstrasse 132, D-5090 Leverkusen 1 (DE)**
Erfinder: **Hagemann, Hermann, Dr., Kandinskystrasse 52, D-5090 Leverkusen 1 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-(2,2,2-Trifluorethyl)-N-alkyl-azolyloxyessigsäureamide, ein Verfahren zu ihrer Herstellung und ihre Verwendung als Herbizide sowie neue α-substituierte N-(2,2,2-Trifluorethyl)-N-alkyl-essigsäureamide als Zwischenprodukte zu ihrer Herstellung.

Es ist bereits bekannt geworden, dass bestimmte substituierte Carbonsäureamide wie z. B. Benzoxazol-2-yl-oxyessigsäure-1, 2, 3, 4–tetrahydroisochinolid, Benzoxazol–2–yl–oxyessigsäure–isobutylamid und 3-Isopropyl-1,2,4-thiadiazol-5-yloxyessigsäure-N-ethyl-N-butyl-amid herbizide Eigenschaften aufweisen (vergleiche die europäischen Patentanmeldungen Nr. 5501 und 18 497).

Die herbiziden Eigenschaften dieser Verbindungen sind jedoch hinsichtlich Wirkungshöhe und Selektivität nicht immer zufriedenstellend.

Es wurden nun neue N-(2,2,2-Trifluorethyl)-N-alkyl-azolyloxyessigsäureamide der Formel I

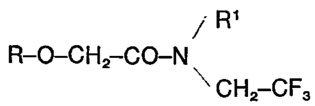
(I)

gefunden, in welcher

R für den Rest

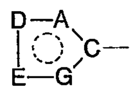

steht, worin

A für C–$R^2$ oder N steht,

D für C–$R^3$ oder N steht,

E für C–$R^4$, N, O oder S steht und

G für C–$R^5$, N, O oder S steht,

mit der Massgabe, dass wenigstens eines der Ringglieder A, D, E oder G für N steht und mindestens eines der Ringglieder E oder G für O oder S steht,

wobei die Reste

$R^2$, $R^3$, $R^4$ und $R^5$, welche gleich oder verschieden sein können, einzeln für Wasserstoff, Halogen, Nitro, Cyano, Amino, $C_1$–$C_4$-Alkylamino, Di-$C_1$–$C_4$-Alkylcarbonylamino, $C_1$–$C_4$-Alkyl-carbonyl, Carboxy, $C_1$–$C_4$-Alkoxycarbonyl, Carbamoyl, $C_1$–$C_4$-Alkylamino-carbonyl, Di-$C_1$–$C_4$-alkylaminocarbonyl, für gegebenenfalls durch Halogen, Nitro oder $C_1$–$C_4$-Alkyl substituiertes Phenylaminocarbonyl, für gegebenenfalls durch Halogen, Nitro, Cyano, $C_1$–$C_4$-Alkyl oder $C_1$–$C_4$-Alkoxy substituiertes Phenyl, für gegebenenfalls durch Halogen substituiertes Benzyl oder Phenylethyl, für gegebenenfalls durch Halogen substituiertes $C_1$–$C_4$-Alkoxy, $C_1$–$C_4$-Alkoxycarbonylmethoxy, Benzyloxy oder Phenoxy, für gegebenenfalls halogen-substituiertes $C_1$–$C_4$-Alkylthio, $C_1$–$C_4$-Alkoxy-carbonylmethylthio, Benzylthio, Phenylthio, $C_1$–$C_4$-Alkylsulfinyl oder $C_1$–$C_4$-Alkylsulfonyl, für gegebenenfalls halogen-substituiertes $C_2$–$C_6$-Alkyl, $C_2$–$C_6$-Alkenyl oder $C_2$–$C_6$-Alkinyl, für Cyano-$C_1$–$C_4$-alkyl, $C_1$–$C_4$-Alkoxy-$C_1$–$C_2$-alkyl, Phenoxy- und Phenylthio-methyl, Benzyloxy- und Benzylthiomethyl, $C_1$–$C_4$-Alkylthio-$C_1$–$C_2$-alkyl, $C_1$–$C_4$-Alkyl- und Phenylsulfinyl-$C_1$–$C_2$-alkyl, $C_1$–$C_4$-Alkyl- und Phenylsulfonyl-$C_1$–$C_2$-alkyl, Carboxy-$C_1$–$C_2$-alkyl, $C_1$–$C_4$-Alkoxy-carbonyl-$C_1$–$C_2$-alkyl, $C_1$–$C_4$-Alkylaminocarbonyl-$C_1$–$C_2$-alkyl, Di-$C_1$–$C_4$-Alkylaminocarbonyl-$C_1$–$C_2$-alkyl, Phenylaminocarbonyl-$C_1$–$C_2$-alkyl oder $C_3$–$C_{12}$-Cycloalkyl stehen,

oder wobei die Reste $R_3$ und $R_4$ zusammen mit den angrenzenden C-Atomen für einen annellierten Benzorest stehen der durch Halogen, Nitro, Cyano oder durch gegebenenfalls halogen-substituierte Reste aus der Reihe $C_1$–$C_4$-Alkyl, $C_1$–$C_4$-Alkoxy, $C_1$–$C_4$-Alkylthio oder $C_1$–$C_2$-Alkylendioxy substituiert sein kann; und worin

$R^1$ für $C_1$–$C_6$-Alkyl steht.

Man erhält die neuen Verbindungen der Formel (I), wenn man N-(2,2,2-Trifluorethyl)-N-alkylhydroxy-essigsäureamide der Formel II

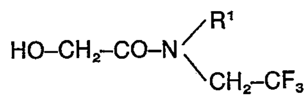
(II)

in welcher

$R^1$ die oben angegebene Bedeutung hat, bzw. deren mit Säurebindemitteln gebildete Salze, mit Halogenhetarenen der Formel III

R–Hal (III)

in welcher

R die oben angegebene Bedeutung hat und

Hal für Chlor oder Brom steht,

gegebenenfalls in Gegenwart eines Säureakzeptors und gegebenenfalls in Gegenwart eines Verdünnungsmittels umsetzt.

Die erfindungsgemässen N-(2,2,2-Trifluorethyl)-N-alkyl-azolyloxyessigsäureamide sind durch Formel (I) definiert. Vorzugsweise steht in dieser Formel R für einen der nachstehenden Azolylreste:

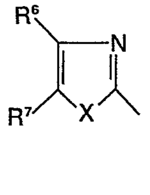 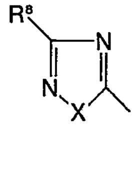 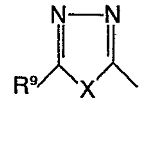 oder 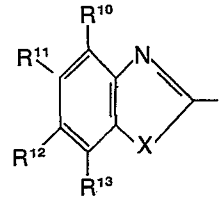

worin

X jeweils für Sauerstoff oder Schwefel steht, die Reste

$R^6$, $R^7$, $R^8$ und $R^9$, welche gleich oder verschieden sein können, einzeln für Wasserstoff, Brom, Chlor, Nitro, Cyano, $C_1$–$C_3$-Alkylcarbonyl, $C_1$–$C_3$-

Alkoxycarbonyl, gegebenenfalls durch Fluor, Chlor oder Brom, Methyl, Methoxy, Nitro, Amino und/oder Cyano 1- oder 2-fach substituiertes Phenyl, für Phenoxy oder Phenylthio, für $C_1$–$C_3$-Alkylthio oder $C_1$–$C_3$-Alkoxy, für $C_1$–$C_3$-Alkylsulfinyl oder $C_1$–$C_3$-Alkylsulfonyl, für $C_1$–$C_4$-Alkyl, Trifluormethyl, Cyano-$C_1$–$C_4$-alkyl, $C_2$–$C_4$-Alkenyl, Benzyloxymethyl, $C_1$–$C_3$-Alkylamino, N-$C_1$–$C_3$-Alkyl-N-$C_1$–$C_4$-alkyl-carbonylamino, Phenoxymethyl-benzylthio, oder für $C_1$–$C_3$-Alkyl-carbonyloxy stehen und die Reste

$R^{10}$, $R^{11}$, $R^{12}$ und $R^{13}$, welche gleich oder verschieden sein können, einzeln für Wasserstoff, Chlor, $C_1$–$C_2$-Alkyl, $C_1C_2$-Alkoxy, Trifluormethyl oder Trifluormethoxy stehen oder jeweils zwei benachbarte Reste zusammen für Methylendioxy, Dichlormethylendioxy oder Difluormethylendioxy stehen; in welcher weiter

$R^1$ für $C_1$–$C_3$-Alkyl steht.

Verwendet man als Ausgangsstoffe beispielsweise N-(2,2,2-Trifluorethyl)-N-propyl-hydroxyessigsäureamid und 2,4,5-Trichlorthiazol, so kann der Reaktionsablauf nach dem erfindungsgemässen Verfahren durch folgendes Formelschema wiedergegeben werden:

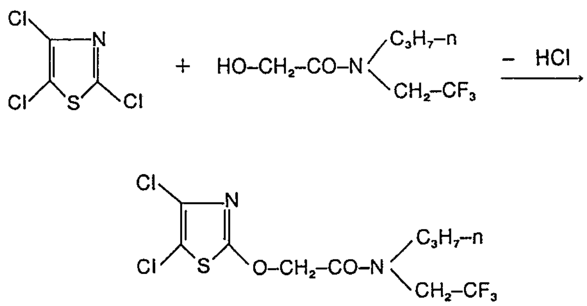


Das Verfahren zur Herstellung der neuen N-(2,2,2-Trifluorethyl)-N-alkylazolyloxyessigsäureamide wird vorzugsweise unter Verwendung geeigneter Verdünnungsmittel durchgeführt. Als solche kommen Wasser und praktisch alle üblichen organischen Solventien infrage. Hierzu gehören insbesondere Alkohole, wie Methanol, Ethanol, n- und iso-Propanol, n-, iso-, sek- und tert-Butanol, Ether wie Diethylether, Dibutylether, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden: hierzu gehören insbesondere Alkali- und Erdalkalihydroxide bzw. -oxide wie Natrium- und Kaliumhydroxid sowie Calciumoxid oder Calcium-hydroxid, Alkali- und Erdalkalicarbonate wie Natrium-, Kalium- und Calciumcarbonat, Alkali-alkoholate wie Natrium-methylat, -ethylat und -tert-butylat, Kaliummethylat, -ethylat und tert-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines grösseren Bereichs variiert werden. Im allgemeinen arbeitet man zwischen −50 und +150 °C, vorzugsweise bei −20 bis +100 °C.

Das erfindungsgemässe Verfahren wird im allgemeinen bei Normaldruck durchgeführt.

Zur Durchführung des erfindungsgemässen Verfahrens werden die Ausgangsstoffe der Formeln (II) und (III) im allgemeinen in angenähert äquimolaren Mengen eingesetzt. Die Komponenten werden gewöhnlich unter leichter Aussenkühlung zusammengegeben und das Reaktionsgemisch wird bis zum Reaktionsende gerührt. Die Isolierung der Produkte erfolgt nach üblichen Methoden: Man destilliert gegebenenfalls einen Teil des Verdünnungsmittels ab, und verdünnt den Rückstand mit Wasser. Soweit die Produkte hierbei kristallin anfallen, werden sie durch Absaugen isoliert. Andernfalls werden die organischen Produkte mit einem mit Wasser nicht mischbaren Lösungsmittel wie z. B. Toluol oder Methylenchlorid extrahiert; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.

Die als Ausgangsstoffe zu verwendenden N-(2,2,2-Trifluormethyl)-N-alkyl-hydroxyessigsäureamide sind durch Formel (II) definiert.

$R^1$ steht darin vorzugsweise für $C_1$–$C_6$-Alkyl, insbesondere für $C_1$–$C_3$-Alkyl.

Als Ausgangsstoffe der Formel (II) seien beispielsweise genannt:
N-(2,2,2-Trifluorethyl)-N-methyl-,
N-(2,2,2-Trifluorethyl)-N-ethyl-
N-(2,2,2-Trifluorethyl)-N-n-propyl- und
N-(2,2,2-Trifluorethyl)-N-iso-propyl-
hydroxyessigsäureamid.

Die N-(2,2,2-Trifluorethyl)-N-alkyl-hydroxyessigsäureamide der Formel (II) sind noch nicht in der Literatur beschrieben. Man erhält die Verbindungen der Formel (II), wenn man entsprechende Acetoxyessigsäureamide der Formel IV

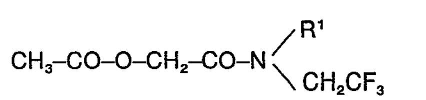 (IV)

in welcher

$R^1$ die oben angegebene Bedeutung hat,

mit wässrig-alkoholischen Alkalilaugen, wie z. B. mit Natriumhydroxid in Wasser/Methanol (1:1), bei Temperaturen zwischen 20 und 80 °C umsetzt.

Zur Aufarbeitung und Isolierung der Produkte wird mit Essigsäure neutralisiert, eingeengt und aus dem Rückstand das Produkt mit Methylenchlorid extrahiert. Nach Trocknen und Filtrieren wird von der Extraktionslösung das Lösungsmittel unter vermindertem Druck sorgfältig abdestilliert. Die Produkte der Formel (II) verbleiben hierbei als öliger oder kristalliner Rückstand.

Die als Zwischenprodukte zu verwendenden N-(2,2,2-Trifluorethyl)-N-alkyl-acetoxyessigsäureamide sind durch Formel (IV) definiert.

$R^1$ steht darin vorzugsweise für $C_1$–$C_6$-Alkyl, insbesondere für $C_1$–$C_3$-Alkyl.

Als Beispiele für die Verbindungen der Formel (IV) seien genannt:
N-(2,2,2-Trifluorethyl)-N-methyl-,
N-(2,2,2-Trifluorethyl)-N-ethyl-,
N-(2,2,2-Trifluorethyl)-N-n-propyl- und
N-(2,2,2-Trifluorethyl)-N-iso-propyl-
acetoxyessigsäureamid.

Die Verbindungen der Formel (IV) sind noch nicht in der Literatur beschrieben. Man erhält sie, wenn man entsprechende Amine der Formel V

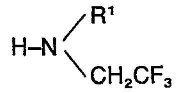 (V)

in welcher

$R^1$ die oben angegebene Bedeutung hat,

mit Acetoxyacetylchlorid der Formel VI

$$CH_3–CO–O–CH_2–CO–Cl \qquad (VI)$$

gegebenenfalls in Gegenwart eines Säurebindemittels, wie z.B. Triethylamin, und gegebenenfalls in Gegenwart eines Verdünnungsmittels, wie z.B. Toluol, bei Temperaturen zwischen 10 und 50 °C umsetzt.

Zur Aufarbeitung wird abgesaugt, vom Filtrat das Lösungsmittel unter vermindertem Druck abdestilliert und das im Rückstand verbleibende Produkt der Formel (IV) gegebenenfalls durch Destillation gereinigt.

Acetoxyacetylchlorid (VI) ist bereits bekannt (vergleiche EP 5501).

Die Amine der Formel (V) sind ebenfalls literaturbekannt bzw. in vorgängigen Patentanmeldungen beschrieben (vergleiche J. Org. Chem. 24 (1959), 1256–1259; DE-A-3 018 020 bzw. EP-A-39 813 sowie DE-A-3 018 030).

Als Beispiele für die Amine der Formel (V) seien N-(2,2,2-Trifluorethyl)-N-methylamin, N-(2,2,2-Trifluorethyl)-N-ethylamin und N-(2,2,2-Trifluorethyl)-N-n-propylamin genannt.

Die weiter als Ausgangsstoffe zu verwendenden Halogenhetarene sind durch Formel (III) definiert. In dieser Formel steht R vorzugsweise bzw. insbesondere für diejenigen Reste, welche bereits im Rahmen der Substituentendefinitionen der Formel (I) vorzugsweise bzw. als insbesondere bevorzugt genannt sind und Hal steht vorzugsweise für Chlor oder Brom.

Als Ausgangsstoffe der Formel (III) seien beispielsweise genannt:
2-Chlor- und 2-Brom-oxazol und -thiazol,
2,4-Dichlor-, 2,5-Dichlor- und
2,4,5-Trichlor-oxazol und -thiazol,
4-Methyl-, 5-Methyl-, 4-tert-Butyl-,
4,5-Dimethyl-, 4-Methyl-5-cyano-,
4-Methyl-5-chlor-, 5-Methyl-4-chlor-,
4-Methyl-5-methoxycarbonyl-,
4-Methyl-5-ethoxycarbonyl-,
4-Methyl-5-isopropoxycarbonyl-,
4-Methyl-5-acetyl-, 5-Phenyl-,
4,5-Diphenyl-, 4-Chlor-5-phenyl-,
4-Chlor-5-(3,4-dichlor-phenyl)- und
4-Methyl-5-phenylthio-, -2-chlor-oxazol,
-2-brom-oxazol, -2-chlor-thiazol und
-2-brom-thiazol; 3-tert-Butyl-4-cyano-,
3-But-3-en-1-yl-3,4-Bis-ethoxycarbonyl-,
3-Phenyl-,
3-Ethyl-4-phenyl-5-chlor-isoxazol,
-5-chlor-isothiazol, -5-brom-isoxazol
und-5-brom-isothiazol;
3,5-Bis-ethoxycarbonyl-4-chlor- und
3,5-Bis-ethoxycarbonyl-4-brom-isoxazol
und-isothiazol;
3,5-Dichlor-1,2,4-oxadiazol, 3-Methyl-,
3-Ethyl-, 3-n-Propyl-, 3-iso-Propyl-,
3-tert-Butyl-, 3-Trifluormethyl-,
3-Trichlormethyl-, 3-Methylthio-,
3-Methylsulfinyl-,
3-Methylsulfonyl-5-chlor-1,2,4-thiadiazol
und-5-brom-1,2,4-thiadiazol;
4-Methyl-, 4-Ethyl-, 4-n-Propyl- und
4-iso-Propyl-, -3-chlor-1,2,5-thiadiazol
und-3-brom-1,2,5-thiadiazol;
2-Chlor- und 2-Brom-1,3,4-oxadiazol,
2-Chlor- und 2-Brom-1,3,4-thiadiazol,
5-Methyl-, 5-ethyl-, 5-n-Propyl-,
5-Phenyl-, 5-iso-Propyl-, 5-tert-Butyl-,
5-Brom-, 5-Methylsulfinyl-,
5-Ethylsulfinyl-, 5-Propylsulfinyl-,
5-Methylsulfonyl-, 5-Ethylsulfonyl-,
5-Propylsulfonyl-, 5-Methoxycarbonyl-,
5-Ethoxy-carbonyl-,
5-(1-Cyano-2-methyl-propyl)-,
5-Benzyloxymethyl-, 5-Acetylamino-,
5-Nitro-, 5-Propylthio-, 5-Trifluormethyl-,
5-Methylamino- und
5-(N-Methyl-N-tert-butylcarbonyl-amino)-
-2-chlor-1,3,4-oxadiazol,
-2-brom-1,3,4-oxadiazol,
-2-chlor-1,3,4-thiadiazol und
-2-brom-1,3,4-thiadiazol;
2-Chlor- und 2-Brom-benzoxazol,
2-Chlor- und 2-Brom-benzthiazol,
5-Methyl-2-chlor-benzoxazol,
2-Chlor-6-ethoxy-benzthiazol,
2,5-Dichlor-benzoxazol,
2,6-Dichlorbenzoxazol,
2-Chlor-6-trifluormethyl-benzthiazol,
2-Chlor-5-trifluormethyloxy-benzthiazol,
2-Chlor-5,6-difluormethylendioxy-benzthiazol,
2,4,6,7-Tetrachlorbenzthiazol,
2-Chlor-4,6-difluor-benzthiazol,
2-Chlor-5-nitro-benzthiazol,
2-Chlor-6-nitro-benzthiazol,
2-Chlor-5-nitro-benzoxazol,
2-Chlor-5-cyano-benzoxazol.

Halogenazole der Formel (III) sind bekannt (vergleiche Elderfield, Heterocyclic Compounds Vol. 5 (1957) S. 298 und S. 452; Vol. 7 (1961), S. 463 und S. 541; Weissberger, The Chemistry of Heterocyclic Compounds, (a) «Five-Membered Heterocyclic Compounds with Nitrogen and Sulfur or Nitrogen, Sulfur and Oxygen» (1952), S. 35 and S. 81, (b) «Five and Six-Membered Compounds with Nitrogen and Oxygen» (1962), S. 5, S. 245 und S. 263; Advances in Heterocyclic Chemistry, Vol. 5 (1965)

S. 119; Vol. 7 (1966), S. 183; Vol. 17 (1974), S. 99 und Vol. 20 (1976) S. 65; Synthesis 1978, 803; Tetrahedron Letters 1968, 829; Chem. Ber. 89 (1956), 1534; 90 (1957), 182; 9 (1959), 1928; J. Org. Chem. 27 (1962), 2589; DE-OS 1 670 706, 1 164 413 und 2 213 865).

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe zeigen neben einer sehr guten Wirkung gegen monokotyle Unkräuter auch eine gute herbizide Wirkung bei dikotylen Unkräutern. Ein selektiver Einsatz der erfindungsgemässen Wirkstoffe ist in verschiedenen Kulturen möglich, vor allem in Baumwolle, Rüben, Sojabohnen und Weizen, ferner in Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweisshydrolysate; als Dispergiermittel kommen in frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-,

Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen oder Stäuben.

Die erfindungsgemässen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,05 und 10 kg Wirkstoff pro Ha, vorzugsweise zwischen 0,1 und 8 kg/ha.

Die erfindungsgemässen Wirkstoffe weisen zum Teil bei bestimmten Anwendungskonzentrationen auch eine wachstumsregulierende Wirkung auf.

Die nachfolgenden Beispiele dienen zur weiteren Erläuterung der Erfindung.

Herstellungsbeispiele

Beispiele zur Herstellung von Verbindungen der Formel (I)

(1)

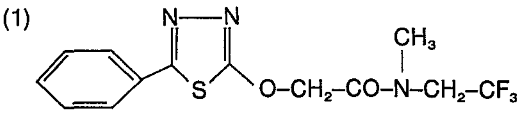

12 g (0,07 Mol) Ny-(2,2,2-Trifluorethyl)-N-methyl-hydroxyessigsäureamid und 4,5 g Kaliumhydroxidpulver werden bei 0 °C in 100 ml Acetonitril dispergiert und bei 10 °C mit einer Lösung von 12 g 2-Chlor-5-phenyl-1,3,4-thiadiazol in 50 ml Acetonitril versetzt. Die Mischung wird 15 Stunden bei 30–40 °C gerührt, eingeengt, mit Wasser verdünnt und abgesaugt. Das kristalline Produkt wird aus Isopropanol umkristallisiert. Man erhält 12 g (63% der Theorie) 5-Phenyl-1,3,4-thiadiazol-2-yl-N-(2,2,2-trifluorethyl)-N-methyl-oxyessigsäureamid vom Schmelzpunkt 123 °C.

(2)

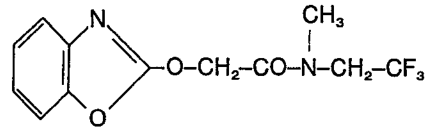

17,1 g (0,1 Mol) N-(2,2,2-Trifluorethyl)-N-methylhydroxyessigsäureamid und 7 g Kaliumhydroxidpulver werden bei 20 °C in 100 ml Acetonitril vorgelegt und bei einer Temperatur zwischen 20 und 40 °C mit 15,3 g (0,1 Mol) 2-Chlor-benzoxazol versetzt. Das Reaktionsgemisch wird eine Stunde bei 40 °C und weitere 15 Stunden bei 20 °C gerührt, eingeengt und mit Toluol und Wasser geschüttelt. Die organische Phase wird mit verdünnter Natronlauge gewaschen, neutral gewaschen, getrocknet und filtriert. Vom Filtrat wird das Lösungsmittel unter vermindertem Druck sorgfältig abdestilliert. Man erhält als Rückstand 15 g (52% der Theorie) vom Schmelzpunkt 92 °C Benzoxazol-2-yl-N-(2,2,2-Trifluorethyl)-N-methyl-oxyessigsäureamid.

Analog Beispiel (1) oder (2) können die in der nachstehenden Tabelle aufgeführten Verbindungen der Formel (I) hergestellt werden:

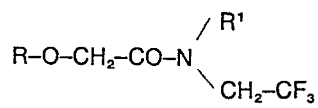

(I)

Tabelle: Verbindungen der Formel (I)

| Verbindungs-Nr. | R | $R^1$ | Schmelzpunkt (°C) bzw. Brechungsindex ($n_D^{20}$) |
|---|---|---|---|
| (3) | 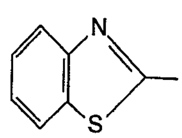 | $CH_3$ | 91 |
| (4) | 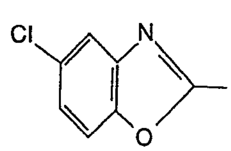 | $CH_3$ | 126 |

Tabelle: (Fortsetzung)

| Verbindungs-Nr. | R | $R^1$ | Schmelzpunkt (°C) bzw. Brechungsindex ($n_D^{20}$) |
|---|---|---|---|
| (5) | 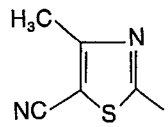 | $CH_3$ | 103 |
| (6) | 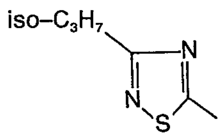 | $CH_3$ | 53 |
| (7) | 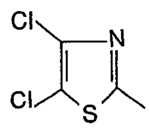 | $CH_3$ | $n_D^{21}$: 1,5088 |
| (8) | 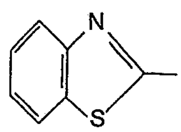 | $C_2H_5$ | 58 |
| (9) | 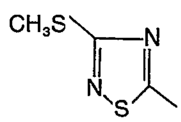 | $CH_3$ | 48 |
| (10) | 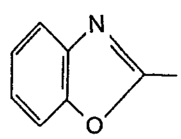 | $C_2H_5$ | 62 |
| (11) | 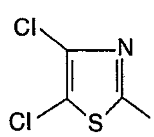 | $C_2H_5$ | |
| (12) | 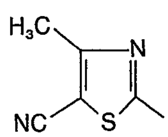 | $C_2H_5$ | 55 |
| (13) | 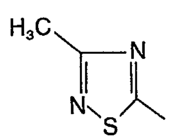 | $CH_3$ | 1,4683 |

Beispiele zur Herstellung von Zwischenprodukten

(a) Verbindungen der Formel (II)

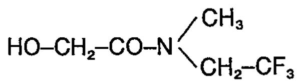

Eine Lösung von 15 g (0,07 Mol) N-(2,2,2-Trifluorethyl)-N-methyl-acetoxyacetamid in 100 ml Methanol wird zu einer Lösung von 3,1 g Natriumhydroxid in 100 ml Wasser gegeben. Das Gemisch wird 8 Stunden bei 40 °C gerührt, dann mit Essigsäure neutralisiert und eingeengt. Es wird dreimal mit Methylenchlorid extrahiert, getrocknet und filtriert. Nach Abdestillieren des Lösungsmittels unter vermindertem Druck verbleiben 5,7 g (47,5% der Theorie) N-(2,2,2-Trifluorethyl)-N-methylhydroxy-essigsäureamid als Öl, welches allmählich kristallisiert: Schmelzpunkt 38 °C.

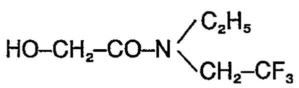

8 ml gesättigte Natronlauge werden zu einer Lösung von 20 g (0,088 Mol) N-(2,2,2-Trifluorethyl)-N-ethyl-acetoxyacetamid in 100 ml Methanol gegeben. Das Gemisch wird 15 Stunden bei 20 °C gerührt, eingeengt, mit Essigsäure neutralisiert und mit Methylenchlorid extrahiert. Die organische Phase wird getrocknet und filtriert. Nach Abdestillieren des Lösungsmittels erhält man N-(2,2,2-Trifluorethyl)-N-ethyl-hydroxyessigsäureamid als gelbes Öl.

(b) Verbindungen der Formel (IV)

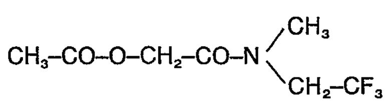

Zu einer Lösung von 9,9 g (0,1 Mol) N-(2,2,2-Trifluorethyl)-N-methylamin in 150 ml Toluol werden 10 g (0,1 Mol) Triethylamin und 13,6 g (0,1 Mol) Acetoxyacetylchlorid gegeben und das Gemisch wird 15 Stunden bei 20 °C geführt. Dann wird abgesaugt und das Filtrat destilliert. Man erhält 17 g (79,8% der Theorie) N-(2,2,2-Trifluorethyl)-N-methyl-acetoxyacetamid vom Siedepunkt 110 °C/3 mbar.

Analog erhält man

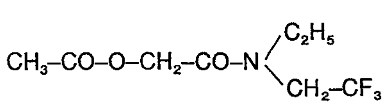

N-(2,2,2-Trifluorethyl)-N-ethyl-acetoxyacetamid; $n_D^{20}$: 1,4333

Beispiel A

Pre-emergence-Test

Lösungsmittel: 5 Gewichtsteile Aceton

Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen z. B. die folgenden Verbindungen gemäss den Herstellungsbeispielen eine ausgezeichnete Wirksamkeit: (2), (4), (5), (6), (7).

**Patentansprüche**

1. N-(2,2,2-Trifluorethyl)-N-alkyl-azolyloxyessigsäureamide der Formel I

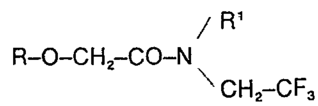 (I)

in welcher

R für den Rest

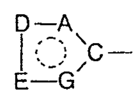

steht, worin

A für C–$R^2$ oder N steht,

D für C–$R^3$ oder N steht,

E für C–$R^4$, N, O oder S steht und

G für C–$R^5$, N, O oder S steht,

mit der Massgabe, dass wenigstens eines der Ringglieder A, D, E oder G für N steht und mindestens eines der Ringglieder E oder G für O oder S steht, wobei die Reste

$R^2$, $R^3$, $R^4$ und $R^5$, welche gleich oder verschieden sein können, einzeln für Wasserstoff, Halogen, Nitro, Cyano, Amino, $C_1$–$C_4$-Alkylamino, Di-$C_1$–$C_4$-Alkylcarbonylamino, $C_1$–$C_4$-Alkyl-carbonyl, Carboxy, $C_1$–$C_4$-Alkoxycarbonyl, Carbamoyl, $C_1$–$C_4$-Alkylamino-carbonyl, Di-$C_1$–C4-alkylaminocarbonyl, für gegebenenfalls durch Halogen, Nitro oder $C_1$–$C_4$-Alkyl substituiertes Phenylaminocarbonyl, für gegebenenfalls durch Halogen, Nitro, Cyano, $C_1$–$C_4$-Alkyl oder $C_1$–$C_4$-Alkoxy substituiertes Phenyl, für gegebenenfalls durch Halogen substituiertes Benzyl oder Phenylethyl, für gegebenenfalls durch Halogen substituiertes $C_1$–$C_4$-Alkoxy, $C_1$–$C_4$-Alkoxycarbonylmethoxy, Benzyloxy oder Phenoxy, für gegebenenfalls halogen-substi-

tuiertes $C_1$–$C_4$-Alkylthio, $C_1$–$C_4$-Alkoxycarbonylmethylthio, Benzylthio, Phenylthio, $C_1$–$C_4$-Alkylsulfinyl oder $C_1$–$C_4$-Alkylsulfonyl, für gegebenenfalls halogen-substituiertes $C_1$–$C_6$-Alkyl, $C_2$–$C_6$-Alkenyl oder $C_2$–$C_6$-Alkinyl, für Cyano-$C_1$–$C_4$-alkyl, $C_1$–$C_4$-Alkoxy-$C_1$–$C_2$-alkyl, Phenoxy- und Phenylthio-methyl, Benzyloxy- und Benzylthiomethyl, $C_1$–$C_4$-Alkylthio-$C_1$–$C_2$-alkyl, $C_1$–$C_4$-Alkyl- und Phenylsulfinyl-$C_1$–$C_2$-alkyl, $C_1$–$C_4$-Alkyl- und Phenylsulfinyl-$C_1$–$C_2$-alkyl, Carboxy-$C_1$–$C_2$-alkyl, $C_1$–$C_4$-Alkoxycarbonyl-$C_1$–$C_2$-alkyl, $C_1$–$C_4$-Alkylaminocarbonyl-$C_1$–$C_2$-alkyl, Di-$C_1$–$C_4$-Alkylaminocarbonyl-$C_1$–$C_2$-alkyl, Phenylaminocarbonyl-$C_1$–$C_2$-alkyl oder $C_3$–$C_{12}$-Cycloalkyl stehen, oder wobei die Reste $R^3$ und $R^4$ zusammen mit den angrenzenden C-Atomen für einen annellierten Benzorest stehen, der durch Halogen, Nitro, Cyano oder durch gegebenenfalls halogen-substituierte Reste aus der Reihe $C_1$–$C_4$-Alkyl, $C_1$–$C_4$-Alkoxy, $C_1$–$C_4$-Alkylthio oder $C_1$–$C_2$-Alkylendioxy substituiert sein kann; und worin

$R^1$ für $C_1$–$C_6$-Alkyl steht.

2. Verfahren zur Herstellung von N-(2,2,2-Trifluorethyl)-N-alkyl-azolyloxyessigsäureamiden der Formel (I) gemäss Anspruch 1, dadurch gekennzeichnet, dass man N-(2,2,2-Trifluorethyl)-N-alkyl-hydroxy-essigsäureamide der Formel II

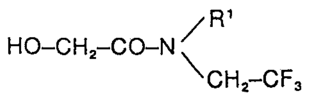

(II)

in welcher

$R^1$ die oben angegebene Bedeutung hat, bzw. deren mit Säurebindemitteln gebildete Salze, mit Halogenhetarenen der Formel III

R–Hal (III)

in welcher

R die oben angegebene Bedeutung hat und

Hal für Chlor oder Brom steht,

gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an mindestens einem N-(2,2,2-Trifluorethyl)-N-alkyl-azolyloxyessigsäureamid der Formel (I) gemäss Anspruch 1.

4. Verfahren zur Bekämpfung von unerwünschtem Pflanzenwachstum, dadurch gekennzeichnet, dass man N-(2,2,2-Trifluorethyl)-N-alkyl-azolyloxyessigsäureamide der Formel (I) gemäss Anspruch 1 auf die unerwünschten Pflanzen oder ihren Lebensraum einwirken lässt.

5. Verwendung von N-(2,2,2-Trifluorethyl)-N-alkyl-azolyloxyessigsäureamiden der Formel (I) gemäss Anspruch 1 zur Bekämpfung von Pflanzenwachstum.

6. Verfahren zur Herstellung von Herbiziden Mitteln, dadurch gekennzeichnet, dass man N-(Trifluorethyl)-N-alkyl-azolyloxyessigsäureamide der Formel (I) gemäss Anspruch 1 mit Streckmitteln und/oder oberflächenaktiven Mitteln vermischt.

7. N-(2,2,2-Trifluorethyl)-N-alkyl-acetoxyessigsäureamide der Formel (IV)

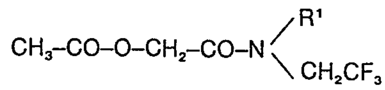

(IV),

in welcher

$R^1$ die in Anspruch 1 angegebene Bedeutung hat.

8. N-(2,2,2-Trifluorethyl)-N-alkyl-hydroxy-essigsäureamide der Formel (II)

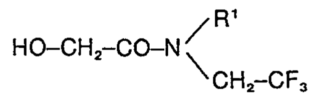

(II),

in welcher

$R^1$ die in Anspruch 1 angegebene Bedeutung hat.

**Claims**

1. N-(2,2,2-Trifluoroethyl)-N-alkyl-azolyloxyacetic acid amides of the formula I

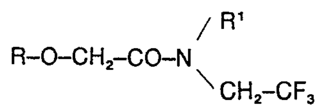

(I)

in which

R represents the radical

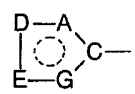

wherein

A represents C–$R^2$ or N,

D represents C–$R^3$ or N,

E represents C–$R^4$, N, O or S and

G represents C–$R^5$, N, O or S,

with the proviso that at least one of the ring members A, D, E or G represents N and at least one of the ring members E or G represents O or S, and wherein

the radicals $R^2$, $R^3$, $R^4$ and $R^5$, which can be identical or different, individually represent hydrogen, halogen, nitro, cyano, amino, $C_1$–$C_4$-alkylamino, di-$C_1$–$C_4$-alkylcarbonylamino, $C_1$–$C_4$-alkylcarbonyl, carboxyl, $C_1$–$C_4$-alkoxy-carbonyl, carbamoyl, $C_1$–$C_4$-alkylamino-carbonyl, di-$C_1$–$C_4$-alkylamino-carbonyl, phenyl-amino-carbonyl which is optionally substituted by halogen, nitro or $C_1$–$C_4$-alkyl, phenyl which is optionally substituted by halogen, nitro, cyano, $C_1$–$C_4$-alkyl or $C_1$–$C_4$-alkoxy, optionally halogen-substituted benzyl or phenethyl, optionally halogen-substituted $C_1$–$C_4$-alkoxy, $C_1$–$C_4$-alkoxycarbonylmethoxy, benzyloxy or phenoxy, optionally halogen-substituted $C_1$–$C_4$-alkylthio, $C_1$–$C_4$-alkoxycarbonyl-methylthio, benzylthio, phenylthio, $C_1$–$C_4$-alkyl-sulphinyl or $C_1$–$C_4$-alkylsulphonyl, optionally halogen-substituted $C_1$–$C_6$-alkyl, $C_2$–$C_6$-alkenyl or $C_2$–$C_6$-alkinyl, or cyano-$C_1$–$C_4$-alkyl, $C_1$–$C_4$-alkoxy-$C_1$–$C_2$-alkyl,

phenoxy- or phenylthiomethyl, benzyloxy- or benzylthio-methyl, $C_1$–$C_4$-alkylthio-$C_1$–$C_2$-alkyl, $C_1$–$C_4$-alkyl- or phenyl-sulphinyl-$C_1$–$C_2$-alkyl, $C_1$–$C_4$-alkyl- or phenyl-sulphonyl-$C_1$–$C_2$-alkyl, carboxy-$C_1$–$C_2$-alkyl, $C_1$–$C_4$-alkoxy-carbonyl-$C_1$–$C_2$-alkyl, $C_1$–$C_4$-alkylamino-carbonyl-$C_1$–$C_2$-alkyl, di-$C_1$–$C_4$-alkylaminocarbonyl-$C_1$–$C_2$-alkyl, phenylaminocarbonyl-$C_1$–$C_2$-alkyl or $C_3$–$C_{12}$-cycloalkyl,
or wherein
the radicals $R^3$ and $R^4$, together with the adjacent C atoms, represent a fused-on-benzo radical, which can be substituted by halogen, nitro, cyano or optionally halogen-substituted radicals from the series comprising $C_1$–$C_4$-alkyl, $C_1$–$C_4$-alkoxy, $C_1$–$C_4$-alkylthio and $C_1$–$C_2$-alkylene-dioxy;
and wherein
$R^1$ represents $C_1$–$C_6$-alkyl.

2. Process for the preparation of N-(2,2,2-trifluoroethyl)-N-alkyl-azolyloxyacetic acid amides of the formula (I) according to Claim 1, characterised in that N-(2,2,2-trifluoroethyl)-N-alkylhydroxy-acetic acid amides of the formula II

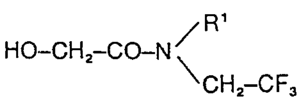
(II)

in which
$R^1$ has the abovementioned meaning,
or salts thereof formed with acid-binding agents, are reacted with halogenohetero-arenes of the formula III

R–Hal (III)

in which
R has the abovementioned meaning and
Hal represents chlorine or bromine,
if appropriate in the presence of an acid acceptor and if appropriate in the presence of a diluent.

3. Herbicidal agents, characterised in that they contain at least one N-(2,2,2-trifluoroethyl)-N-alkyl-azolyl-oxyacetic acid amide of the formula (I) according to Claim 1.

4. Process for combating undesired plant growth, characterised in that N-(2,2,2-trifluoroethyl)-N-alkyl-azolyloxyacetic acid amides of the formula (I) according to Claim 1 are allowed to act on the undesired plants or their environment.

5. Use of N-(2,2,2-trifluoroethyl)-N-alkyl-azolyloxyacetic acid amides of the formula (I) according to Claim 1 for combating plant growth.

6. Process for the preparation of herbicidal agents, characterised in that N-(trifluoroethyl)-N-alkyl-azolyloxyacetic acid amides of the formula (I) according to Claim 1 are mixed with extenders and/or surface-active agents.

7. N-(2,2,2-trifluoroethyl)-N-alkyl-acetoxyacetic acid amides of the formula (IV)

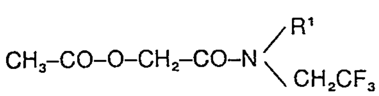
(IV)

in which
$R^1$ has the meaning given in Claim 1.

8. N-(2,2,2-trifluoroethyl)-N-alkyl-hydroxy-acetic acid amides of the formula (II)

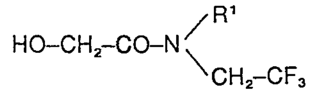
(II)

in which
$R^1$ has the meaning given in Claim 1.

**Revendications**

1. N-(2,2,2-trifluoréthyl)-N-alkylamides d'acides azolyloxyacétiques de formule I

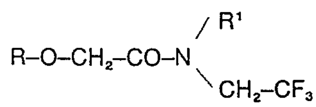
(I)

dans laquelle
R représente le reste

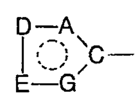

où
A représente C–$R^2$ ou N,
D représente C–$R^3$ ou N,
E représente C–$R^4$, N, O ou S et
G représente C–$R^5$, N, O ou S,
à condition qu'au moins l'un des maillons A, D, E et G du noyau représente N et qu'au moins l'un des maillons E et G du noyau représente O ou S, les restes
$R^2$, $R^3$, $R^4$ et $R^5$, qui peuvent être identiques ou différents, représentent individuellement l'hydrogène, un halogène, un groupe nitro, cyano, amino, alkylamino en $C_1$ à $C_4$, di-(alkyle en $C_1$ à $C_4$)carbonylamino, (alkyle en $C_1$ à $C_4$)-carbonyle, carboxy, (alkoxy en $C_1$ à $C_4$)-carbonyle, carbamoyle, (alkyle en $C_1$ à $C_4$)-amino-carbonyle, di(alkyle en $C_1$ à $C_4$)-aminocarbonyle, un groupe phénylaminocarbonyle éventuellement substitué par un halogène, un groupe nitro ou un groupe alkyle en $C_1$ à $C_4$, un groupe phényle éventuellement substitué par un halogène, un radical nitro, cyano, alkyle en $C_1$ à $C_4$ ou alkoxy en $C_1$ à $C_4$, un groupe benzyle ou phényléthyle éventuellement substitué par un halogène, un groupe alkoxy en $C_1$ à $C_4$, (alkoxy en $C_1$ à $C_4$)carbonylméthoxy, benzyloxy ou phénoxy éventuellement substitué par un halogène, un groupe alkylthio en $C_1$ à $C_4$, (alkoxy en $C_1$ à $C_4$)carbonylméthylthio, benzylthio, phénylthio, (alkyle en $C_1$ à $C_4$)sulfinyle ou (alkyle en $C_1$ à $C_4$)sulfonyle éventuellement substitué par un halogène, un groupe alkyle en $C_1$ à $C_6$, alcényle en $C_2$ à $C_6$ ou alcynyle en $C_2$ à $C_6$ éventuellement substitué par un halogène, un groupe cyano-(alkyle en $C_1$ à $C_4$), (alkoxy en $C_1$à $C_4$)-alkyle en $C_1$ ou $C_2$, phénoy- et phénylthiométhyle, benzyloxy- et benzylthiométhyle, (alkylthio en $C_1$ à $C_4$)-alkyle en $C_1$ ou $C_2$, (alkyle en $C_1$ à $C_4$)- et phénylsulfinyle-(alkyle en $C_1$ ou $C_2$), (alkyle en $C_1$ à $C_4$)- et phénylsulfonyl- (alkyle en $C_1$ ou $C_2$), carboxy- (alkyle en $C_1$ ou $C_2$), (alkoxy en $C_1$ à $C_4$)-carbonyl-(alkyle en $C_1$ ou $C_2$),

(alkyle en $C_1$ à $C_4$)-aminocarbonyl-(alkyle en $C_1$ ou $C_2$), di(alkyle en $C_1$ à $C_4$)aminocarbonyl-alkyle en $C_1$ ou $C_2$, phénylaminocarbonyl-(alkyle en $C_1$ ou $C_2$) ou cycloalkyle en $C_3$ à $C_{12}$,

ou bien les restes

$R^3$ et $R^4$ forment conjointement avec les atomes contigus de carbone un reste benzénique condensé qui peut être substitué par un halogène, un groupe nitro, cyano ou des restes éventuellement substitués par des halogènes, de la série alkyle en $C_1$ à $C_4$, alkoxy en $C_1$ à $C_4$, alkylthio en $C_1$ à $C_4$ ou alkylènedioxy en $C_1$ ou $C_2$;

et

$R^1$ est un groupe alkyle en $C_1$ à $C_6$.

2. Procédé de production de N-(2,2,2-trifluoréthyl)-N-alkylamides d'acides azolyloxyacétiques de formule (I) suivant la revendication 1, caractérisé en ce qu'on fait réagir des N-(2,2,2-trifluoréthyl)-N-alkylamides d'acides hydroxyacétiques de formule II

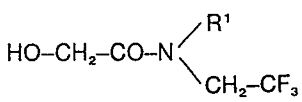

(II)

dans laquelle

$R^1$ a la définition indiquée ci-dessus

ou leurs sels formés avec des accepteurs d'acides, avec des halogénhétarènes de formule III

R–Hal (III)

dans laquelle

R a la définition indiquée ci-dessus et

Hal représente du chlore ou du brome,

éventuellement en présence d'un accepteur d'acide et le cas échéant, en présence d'un diluant.

3. Compositions herbicides, caractérisées par une teneur en au moins un N-(2,2,2-trifluoréthyl)-N-alkylamide d'acide azolyloxyacétique de formule (I) suivant la revendication 1.

4. Procédé pour combattre une croissance indésirable de plantes, caractérisé en ce qu'on fait agir des N-(2,2,2-trifluoréthyl)-N-alkylamides d'acides azolyloxyacétiques de formule (I) suivant la revendication 1 sur les plantes indésirables ou sur leur milieu.

5. Utilisation de N-(2,2,2-trifluoréthyl)-N-alkylamides d'acides azolyloxyacétiques de formule (I) suivant la revendication 1 pour combattre une végétation.

6. Procédé de préparation de compositions herbicides, caractérisé en ce qu'on mélange des N-(trifluoréthyl)-N-alkylamides d'acides azolyloxyacétiques de formule (I) suivant la revendication 1 avec des diluants et/ou des agents tensio-actifs.

7. N-(2,2,2-trifluoréthyl)-N-alkylamides d'acide acétoxyacétique de formule (IV)

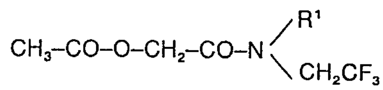

(IV)

dans laquelle

$R^1$ a la définition indiquée dans la revendication 1.

8. N-(2,2,2-trifluoréthyl)-N-alkylamides d'acide hydroxyacétique de formule (II)

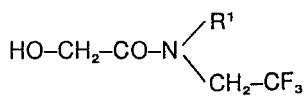

(II)

dans laquelle

$R^1$ a la définition indiquée dans la revendication 1.